# FASCICULE DE BREVET EUROPEEN

(11) **EP 2 445 516 B1**
(45) Date de publication et mention de la délivrance du brevet: **12.08.2015**
(21) Numéro de dépôt: 10734272.7
(22) Date de dépôt: 18.06.2010
(51) Int. Cl.: A61K 38/48, A61K 47/00, A61P 7/04

(54) **COMPOSITION DE FACTEUR VII**
FAKTOR-VII-ZUSAMMENSETZUNG
FACTOR VII COMPOSITION

(30) Priorité: 26.06.2009 FR 0954390
(43) Date de publication de la demande: 02.05.2012
(60) Demande divisionnaire: 15175122.9
(73) Titulaire: Laboratoire Français du Fractionnement et des Biotechnologies, 91940 Les Ulis (FR)
(72) Inventeur: BARDAT, Annie, F-91470 Limours (FR); POMPE, Cornelius, F-92160 Antony (FR)
(74) Mandataire: Chajmowicz, Marion
(86) Numéro de dépôt international: PCT/FR2010/051229
(87) Numéro de publication internationale: WO 2010/149907

(56) Documents cités:
- WO-A1-01/12653
- WO-A1-2008/113589
- WO-A2-2005/016365
- WO-A2-2006/089954
- WO-A2-2007/022784

## Description

La présente invention concerne une composition pharmaceutique stable comprenant du facteur VII (FVII).

### Arrière-plan technologique :

Le phénomène de coagulation comporte une cascade de réactions enzymatiques impliquant les facteurs de la coagulation dont plusieurs sont des protéases comportant une sérine au niveau de leur site actif. L'étape finale est la transformation du fibrinogène soluble en filaments de fibrine qui encerclent dans leurs mailles les cellules circulantes. Les facteurs de la coagulation sont désignés par des numéros allant de I à XIII. A l'exception du facteur XIII qui intervient dans la dernière étape de la coagulation, les autres facteurs interviennent dans l'ordre inverse de leur numérotation ; ainsi le facteur XII initie la coagulation et le facteur 1 la termine. Chaque facteur existe sous forme de précurseur inactif et sous forme activée, indiquée par la lettre a.

La coagulation met en jeu deux voies, l'une intrinsèque, l'autre extrinsèque, aboutissant à une voie finale commune. La combinaison des deux mécanismes assure la formation d'un caillot de sang solide et flexible, qui résiste à la pression sanguine tout en garantissant une mobilité suffisante. Sous l'action de la thrombine, le fibrinogène subit des modifications chimiques qui aboutissent à la formation de la fibrine. La fibrine est nécessaire à la formation du caillot.

La voie intrinsèque comporte les facteurs présents dans la circulation et le processus de coagulation démarre au sein même du vaisseau sanguin. La voie extrinsèque quant à elle, met en jeu les facteurs tissulaires non présents normalement dans la circulation et qui sont libérés lors d'une lésion vasculaire. C'est lors de l'activation de cette voie que se produit une réaction en chaîne, au cours de laquelle un facteur de coagulation activé enclenche l'activation du facteur de coagulation suivant. Cette voie implique l'intervention du facteur VII (FVII) présent dans le plasma. Le facteur VII activé, également appelé proconvertine, est un des facteurs de poids moléculaire d'environ 50 kDa, impliqué dans le mécanisme de la coagulation sanguine. C'est une glycoprotéine de la famille des sérines protéases, dont la synthèse sous forme activée est dépendante de la vitamine K. Pour initier la cascade de coagulation, le FVII doit être activé en FVIIa. Le FVIIa seul (non complexé) présente une faible activité protéolytique. Puis une fois activé, le FVIIa se complexe au facteur tissulaire (FT), protéine associée à des phospholipides, qui est libérée lors de la lésion vasculaire. Le complexe FVIIa-FT transforme ensuite le facteur X en facteur Xa en présence d'ions calcium. Ce complexe agit également sur l'activation du facteur FIX en FIXa, catalysant ainsi la voie intrinsèque. Les facteurs IXa et Xa, activent en retour le facteur VII activé. Le facteur Xa complexé au facteur FV activé et à la prothrombinase, transforme la prothrombine en thrombine. La thrombine agit alors sur le fibrinogène pour le transformer en fibrine et permet également l'activation du FVIIIa et du FVa à partir respectivement de FVIII et du FV. La thrombine permet pour sa part, lorsqu'elle est en présence de calcium, d'activer le facteur XIIIa responsable de la consolidation du caillot de fibrine. Néanmoins, lorsqu'un facteur de la coagulation fait défaut, la cascade de réactions est interrompue ou déficiente et on parle alors d'une coagulation anormale.

Le facteur VII activé agit localement en présence de facteur tissulaire libéré après une lésion de tissus engendrant des hémorragies, même en l'absence de facteur VIII ou IX. C'est pourquoi le facteur VII, de préférence sous forme activée, est utilisé depuis longtemps pour le traitement de certains troubles de la coagulation sanguine se manifestant par des saignements. Le facteur est impliqué dans de nombreuses pathologies, comme l'hémophilie de type A ou B dont les patients présentent des inhibiteurs des facteurs VIII ou IX, l'hémophilie acquise, le déficit congénital en facteur VII et en tant que produit pour la prévention des hémorragies pouvant avoir lieu lors d'opérations chirurgicales.

A l'heure actuelle, on connait un médicament disponible sur le marché pour le traitement de ces patients souffrant d'hémophilie ou de déficit congénital en facteur VII. Il s'agit du NovoSeven® autorisé sur le marché européen depuis 1996 et autorisé sur le marché américain en 1999, produit par la firme danoise NovoNordisk. Le NovoSeven® est un médicament dont le principe actif est l'eptacog alfa (facteur VII activé de la coagulation recombinant humain activé produit par génie génétique à partir de cellules rénales BHK de hamsters nouveau-nés). Ce produit contient en outre du chlorure de sodium (2,92 g/l), du chlorure de calcium dihydraté (1,47 g/l), de la glycylglycine (1,32 g/l), du polysorbate 80 (0,07 g/l), de mannitol (30 g/l).

Il existe également une variante du NovoSeven intitulé NovoSeven® RT, qui permet une conservation du produit à température ambiante (25°C). Ce second produit est constitué de chlorure de sodium (2,92 g/l), de chlorure de calcium dihydraté (1,47 g/l), de glycylglycine (1,32 g/l), de polysorbate 80 (0,07 g/l), de mannitol (25 g/l), d'acide chlorhydrique et d'hydroxyde de sodium pour l'ajustement du pH, et contient également du saccharose (10 g/l) et de la méthionine (0,5 g/l) (utilisée comme antioxydant). La remise en solution de ce produit nécessite de l'eau pour préparation injectable mais également de l'histidine. Le NovoSeven® RT a été autorisé sur les marchés européen et américain en 2008.

La principale indication thérapeutique pour le FVIIa recombinant (rFVIIa) concerne le traitement du saignement spontané ou chirurgical des hémophiles A ayant développé des anticorps anti-facteur VIII et des hémophiles B ayant développé des anticorps anti-facteurs IX. En Europe, il est aussi indiqué pour son utilisation chez des patients avec une déficience congénitale en FVII et chez les patients atteints de la thrombasthénie de Glanzmann. En outre, de nombreuses publications rapportent l'efficacité du rFVIIa dans le contrôle de l'hémorragie lors d'interventions chirurgicales, chez des patients qui n'ont ni déficit congénital en facteur de la coagulation ni de thrombasthénie.

Un article de Nedergaard et al, 2008 [Nedergaard H. et al, In vitro stability of lyophilized and reconstituted recombinant activated factor VII formulated for storage at room temperature, Clinical Therapeutics, Vol 30, N°7, p1309-1315, 2008] enseigne que le NovoSeven® RT reste stable durant une période de 24 mois à 25°C, 12 mois à 30°C, 6 mois à 40°C et 12 heures à 50°C et 60 °C sous sa forme lyophilisée. De plus, ce produit n'est stable que pendant six heures après sa reconstitution liquide, et par conséquent il est recommandé de réaliser l'injection dans les trois heures suivant la reconstitution. Ce produit présente donc, par cette stabilité, des difficultés de manipulation et des contraintes au niveau des horaires d'administration.

Le brevet EP 1 210 361 divulgue l'intérêt d'utiliser de la glycylglycine pour stabiliser une composition lyophilisée de facteur VII activé.

La demande de brevet WO2004/000347 propose divers autres agents de stabilisation.

La publication de Soenderkaer S. et al, 2004 [Soenderkaer S. et al, Effects of sucrose on rFVIIa aggregation and methionine oxidation, European Journal of Pharmaceutical Sciences, Vol 21, p597-606, 2004] décrit le saccharose comme excipient essentiel pour la stabilisation contre l'agrégation et la dénaturation thermique du facteur VII activé. Selon cet enseignement, le saccharose permet à la protéine de conserver sa forme native en solution aqueuse, en excluant les sucres de la surface des protéines, ce qui permet d'augmenter le potentiel chimique de la molécule. Par conséquence et en réduisant sa surface, la protéine reste dans une conformation compacte. La présence de saccharose en tant qu'excipient permet de stabiliser le facteur VII activé sous sa forme lyophilisée. Cependant, la présence de saccharose a pour conséquence l'induction de composés antioxydants dans la formulation, conduisant à des contraintes techniques et réglementaires associées à l'ajout de ce type de composés.

Il existe donc aujourd'hui un réel besoin de développer des médicaments contenant du facteur VII, dépourvus d'antioxydants, stables chimiquement et physiquement à température ambiante, et faciles à utiliser par des patients notamment atteints d'hémophilie ou de déficit congénital en facteur VII.

### Résumé de l'invention :

L'invention fournit une composition pharmaceutique sous forme liquide ou sous forme solide, comprenant du facteur VII, de préférence sous forme de facteur VIIa, ladite composition étant dépourvue de mannitol et de saccharose.

Cette composition comprend
- arginine, éventuellement sous forme de chlorhydrate ;
- isoleucine ;
- lysine , éventuellement sous forme de chlorhydrate ;
- glycine ;
- citrate trisodique ou chlorure de calcium ;
- et le cas échéant du polysorbate 80 ou polysorbate 20.

Selon un mode de réalisation préféré, la composition est en outre dépourvue de tout agent antioxydant.

De manière avantageuse, la composition sous forme solide peut être sous forme d'une poudre ou d'un gâteau (« cake » ou « plug »). La composition présente de préférence un degré d'humidité inférieur ou égal à 3%. Selon un mode de réalisation particulier, la composition est sous forme lyophilisée.

Un autre objet de l'invention est un procédé de préparation d'une telle composition, ledit procédé comprenant le mélange du FVII avec une solution tampon, ajustement du pH si nécessaire, filtration pour obtenir une forme liquide. Cette forme liquide peut ensuite subir une dessiccation pour obtenir la forme solide.

Le terme « solution tampon » inclut au moins un acide aminé hydrophile ou portant une chaîne latérale chargée positivement, qui est l'arginine, et un sel de métal alcalin, un sel de métal alcalino-terreux, ou un sel d'un métal de transition, qui est du citrate trisodique ou chlorure de calcium.

Un autre objet de l'invention consiste en un procédé de préparation d'une formulation injectable à usage thérapeutique, ledit procédé comprenant la dissolution d'une composition solide telle que définie ici, dans de l'eau pour préparations injectables.

L'invention vise également une formulation injectable susceptible d'être obtenue par ce procédé.

### Description détaillée de l'invention :

La demanderesse fournit des compositions de facteur VII stables chimiquement et physiquement à température ambiante, et faciles à utiliser par des patients atteints d'hémophilie ou de déficit congénital en facteur VII, notamment.

Notamment, la nouvelle composition pharmaceutique de l'invention présente une stabilité sous forme solide supérieure à 24 mois à une température inférieure ou égale à 25°C.

La composition peut donc être conservée à température ambiante sans dégradation substantielle du facteur VII. Par température ambiante, on entend la température à l'intérieur d'une pièce, normalement comprise entre 10°C to 30°C, de préférence entre 15°C to 25°C.

Le terme "Facteur VII " ou "FVII" inclut les polypeptides comprenant la séquence 1-406 du facteur VII humain de type sauvage (comme décrit dans le brevet U. S. 4,784, 950), ou du FVII dérivé d'une autre espèce (par exemple bovin, porcin, canin, murin). Il comprend en outre les variations alléliques naturelles du facteur VII qui peuvent exister, et toute forme ou degré de glycosylation ou autre modification post-traductionnelle.

Le terme « Facteur VII » inclut aussi les variants du FVII qui présente la même ou une activité biologique supérieure par rapport à l'activité de la forme sauvage, ces variants incluant notamment les polypeptides différant du FVIIa sauvage par insertion, délétion, ou substitution d'un ou plusieurs acides aminés.

Le « Facteur VII » ou « FVII » comprend le FVII non clivé (zymogène) et le Facteur VII activé. Le facteur VII est utilisé dans la composition de préférence sous sa forme activée.

Le terme "activité biologique du facteur VIIa" inclut la capacité à générer de la thrombine, par exemple à la surface des plaquettes activées. L'activité du Facteur VII dans la composition peut être évaluée de différentes manières. Par exemple, elle peut être mesurée par le rapport entre la quantité de Facteur VIIa déterminée par un test de coagulation et la quantité de Facteur VII déterminée par immunoréactivité avec des anticorps anti-FVII.

Le terme "composition stable" signifie ici que la formation d'agrégats (insolubles ou solubles) est minimisée, et/ou que la dégradation chimique est réduite, le pH est maintenu et la conformation de la protéine n'est pas substantiellement modifiée pendant la production ou la conservation des compositions de l'invention, de telle sorte que l'activité biologique et la stabilité de la protéine soient conservées. Lorsque les compositions sont soumises à une lyophilisation, la stabilisation des compositions impliquent une lyoprotection et une cryoprotection de la protéine.

Le terme "stabilité physique" du Facteur VII se réfère à la réduction ou l'absence de formation d'agrégats insolubles ou solubles des formes dimériques, oligomériques ou polymériques du Facteur VII, ainsi qu'à la réduction ou l'absence de toute dénaturation structurale de la molécule.

Le terme "stabilité chimique" se réfère à la réduction ou l'absence de toute modification chimique du facteur VII pendant le stockage, à l'état solide ou sous forme dissoute, dans des conditions accélérées. Par exemple, les phénomènes d'hydrolyse, déamination, et/ou oxydation sont évités ou retardés. L'oxydation des acides aminés contenant du soufre est limitée.

Par exemple, les compositions solides de l'invention contiennent de faibles teneurs en formes oxydées et en agrégats à la fin du procédé de préparation et avant conservation, par exemple moins de 5% en poids, de préférence moins de 4%, ou encore moins de 3% ou moins de 2% en poids de FVII est converti en forme oxydée, et moins de 5% en poids, de préférence moins de 4%, ou encore moins de 3% ou moins de 2% en poids de FVII est converti en forme dimérique ou polymérique. Lors de la conservation, de préférence moins de 10% en poids de FVII est converti en forme oxydée, et moins de 10% en poids de FVII est converti en forme dimérique ou polymérique, après conservation à 30°C après 24 mois à l'obscurité.

Une composition lyophilisée selon l'invention présente aussi une stabilité structurale, c'est-à-dire qu'elle est capable de former un gâteau (« cake » ou « plug ») qui ne s'effrite pas spontanément, et qui est facilement soluble dans de l'eau avant utilisation.

La composition pharmaceutique de l'invention est une composition sous forme liquide ou sous forme solide, comprenant du facteur VII, ladite composition étant dépourvue de mannitol et de saccharose.

La teneur en facteur VII, de préférence sous forme de facteur VIIa, dans la composition de l'invention peut être la suivante : entre 0,1 et 15mg/ml, de préférence entre 0,1 et 10mg/ml, de préférence encore entre 0,2 et 5mg/ml, ou encore entre 0,2 et 2mg/ml (mesurée sous forme liquide, de préférence avant dessication, ou éventuellement après reconstitution sous forme de préparation injectable).

De préférence, la composition est dépourvue de tout sucre, de polyol ou de méthionine.

Les sucres à éviter incluent notamment, en plus du saccharose, les di- et tri-saccharides et les polysaccharides, tels que le dextrose, le lactose, le maltose, le trehalose, les cyclodextrines, les maltodextrines et les dextrans.

Les polyols à éviter incluent notamment, en plus du mannitol, le sorbitol et le xylitol.

De préférence encore, la composition est dépourvue de glycylglycine.

Selon un mode de réalisation préféré, la composition de l'invention est en outre dépourvue de tout agent antioxydant. Les antioxydants incluent par exemple un ou plusieurs des composés suivants : homocystéine, cystéine, cystathionine, méthionine, gluthation.

La composition selon l'invention comprend au moins un acide aminé hydrophile ou portant une chaîne latérale chargée positivement, qui est l'arginine, ou un de ses sels dérivés comme l'arginine chlorhydrate ou encore l'arginine phosphate.

Des acides aminés comme la glycine et/ou la lysine, ou un de ses sels dérivés comme la lysine chlorhydrate peuvent être avantageusement ajoutés.

L'addition d'un acide aminé hydrophile ou portant une chaîne latérale chargée positivement tel que l'arginine, et le cas échéant d'un acide aminé hydrophobe voire d'un sel de métal alcalin, alcalino-terreux, ou d'un métal de transition, favorise la stabilisation du facteur VII et la solubilisation des formes lyophilisées.

L'acide aminé hydrophobe utilisé dans le cadre de cette invention est l'isoleucine.

La composition de l'invention comprend un sel de métal alcalin, un sel de métal alcalino-terreux, ou un sel d'un métal de transition, à savoir du citrate de sodium ou du chlorure de calcium.

Enfin, la composition de l'invention peut comprendre un ou plusieurs détergents de type non ionique tels que les polysorbates, poloxamères, polyoxyéthylène alkyl éthers, un bloc co-polymères d'éthylène/polypropylène et polyéthylèneglycol. Avantageusement, les détergents préférés sont le polysorbate 80 et le polysorbate 20.

Dans un exemple de réalisation, la composition comprend :
- facteur VII, de préférence sous forme de facteur VIIa;
- arginine, éventuellement sous forme de chlorhydrate ;
- isoleucine ;
- lysine;
- glycine ;
- citrate trisodique ou chlorure de calcium ;
- et le cas échéant du polysorbate 80 ou polysorbate 20.

Plus particulièrement, la composition peut comprendre
- facteur VII, de préférence sous forme de facteur VIIa ;
- de 10 à 40 g/l d'arginine, éventuellement sous forme de chlorhydrate ;
- de 4,2 à 6,6 g/l d'isoleucine ;
- de 0,6 à 1,8 g/l de lysine ;
- de 0,6 à 1,8 g/l de glycine ;
- de 1 à 2 g/l de citrate trisodique dihydraté ou de 0 à 0,2 g/L de chlorure de calcium dihydraté;
- et le cas échéant de 0 à 0,5 g/l de polysorbate 80.

Selon un exemple particulier, la composition comprend
- du facteur VII (de préférence sous forme de facteur VIIa) à 0,2 à 2g/l,
- du chlorhydrate d'arginine à 24 g/l,
- de l'isoleucine à 6g/l,
- du citrate trisodique dihydraté à 1,5g/l,
- de la glycine à 1,2g/l,
- du chlorhydrate de lysine à 1,2g/l.
- et /ou du polysorbate 80 à 0,07g/l.

Selon un autre exemple particulier, la composition comprend
- facteur VII (de préférence sous forme de facteur VIIa) à 0,2 à 2g/l,
- du chlorhydrate d'arginine à 34 g/l,
- du chlorure de calcium dihydraté à 0,15 g/l,
- de l'isoleucine à 6g/l.

Les concentrations sont déterminées vis à vis des compositions sous forme liquide, avant dessiccation, ou après reconstitution sous forme de préparation injectable.

La Demanderesse a éliminé de sa composition à usage pharmaceutique, le saccharose ainsi que le mannitol, ces ingrédients étant pourtant utilisés de manière usuelle comme diluants ou stabilisants des formulations pharmaceutiques comprenant du facteur VII.

L'absence de saccharose et de mannitol offre plusieurs avantages. D'une part la présence d'éléments oxydants ou d'endotoxines, qui peuvent être apportés par le saccharose, est évitée.

Il est apparu de façon surprenante que l'absence de mannitol permet d'éviter la formation de formes polymorphes des cristaux de mannitol au sein de la composition au cours de lyophilisation, et limite le risque d'impuretés, comme notamment la présence de mannose dans la composition.

Par ailleurs, contre toute attente, l'absence de mannitol et de saccharose de la composition de l'invention ne nuit pas à la stabilité de la composition. Au contraire, on observe une augmentation de la température de transition vitreuse de la composition, par rapport aux formulations disponibles, par exemple le NovoSeven® RT.

La transition vitreuse est une transition du second ordre c'est-a-dire une transition thermique qui implique un changement de capacité calorifique, mais pas de chaleur latente. Elle est caractéristique des liquides surfondus qui sont refroidis à une température suffisamment basse et suffisamment rapidement sans cristalliser et qui deviennent un verre et des polymères amorphes ou de la partie amorphe des polymères cristallins qui passent d'un état solide à un état viscoélastique. La température de transition vitreuse ou Tg est la température à laquelle a lieu ce changement d'état. Quand un produit liquide est refroidi en-dessous de cette température il devient solide et cassant, comme le verre, on dit qu'il est à l'état vitreux.

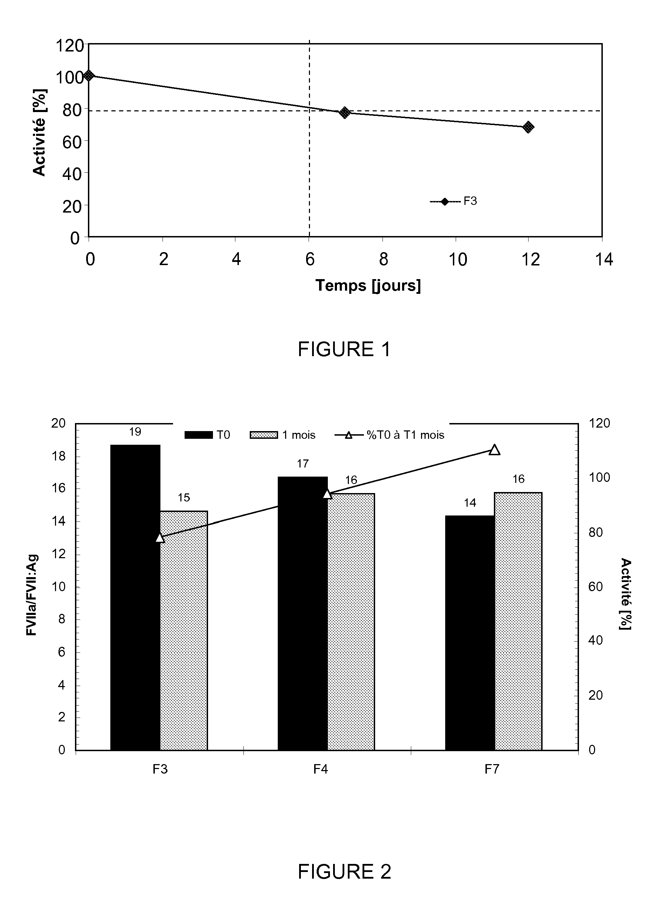

En effet, en présence de mannitol et de saccharose comme dans la formulation de NovoSeven®RT, la température de transition vitreuse est de 45°C. La température de transition vitreuse de la composition de l'invention, exempte de saccharose et de mannitol, est supérieure à 60°C, et généralement comprise entre 74 et 93°C ce qui permet d'envisager un stockage hors réfrigérateur et même à des températures supérieure à 25°C (cf Figure 2).

Le facteur VII est généralement un facteur VII humain. Il peut être obtenu de différentes manières, par exemple à partir de la fraction non cryoprécipitable du plasma humain, ou par génie génétique à partir de cellules, ou encore à partir d'animaux transgéniques.

De manière préférée, le facteur VII (de préférence sous forme de facteur VIIa) est produit notamment dans le lait d'animal transgénique, la formulation de l'invention permettant de conserver au facteur VII une activité biologique satisfaisante après sa lyophilisation.

Selon un mode de réalisation préféré, le facteur VII humain est produit dans le lait de mammifères transgéniques non humains, modifiés génétiquement pour produire cette protéine. De préférence il s'agit du lait d'une lapine ou d'une chèvre transgénique.

La sécrétion de facteur VII par les glandes mammaires, permettant sa sécrétion dans le lait du mammifère transgénique, implique le contrôle de l'expression du facteur VII de manière tissu-dépendante. De telles méthodes de contrôle sont bien connues de l'homme du métier. Le contrôle de l'expression est effectué grâce à des séquences permettant l'expression de la protéine vers un tissu particulier de l'animal. Il s'agit notamment des séquences promotrices WAP, béta-caséine, béta-lactoglobuline et des séquences peptides signal. Le procédé d'extraction de protéines d'intérêt à partir du lait d'animaux transgénique est décrit dans le brevet EPO 264 166.

La composition de l'invention peut être obtenue en utilisant toute technique usuelle.

Notamment la composition de l'invention peut être obtenue en mettant en oeuvre un procédé comprenant le mélange du facteur VII avec une solution tampon, ajustement du pH si nécessaire, filtration pour l'obtention d'une forme liquide, puis dessiccation si nécessaire pour l'obtention d'une forme solide.

De préférence le pH de la solution avant dessiccation est compris entre 4,0 et 9,0, plus particulièrement dans les domaines 4,0 et 8,0 ; 4,0 et 7,5 ; 4,5 et 7,5 ; 5,0 et 7,5 ; 5,5 et 7,0 ; 6,0 et 7,5 ; 6,5 et 7,5

La dessiccation est un procédé d'élimination de l'eau à un stade poussé. Il s'agit d'une déshydratation visant à éliminer autant d'eau que possible. Ce phénomène peut être naturel ou forcé. Cette dessiccation peut être réalisée à l'aide des techniques de lyophilisation, d'atomisation et de cryoatomisation. Le mode préféré d'obtention de la forme solide de la composition à usage pharmaceutique selon l'invention est la lyophilisation.

Les méthodes de lyophilisation sont bien connues de l'homme du métier, voir par exemple [Wang et al, Lyophilization and development of solid protein pharmaceuticals, International Journal of Pharmaceutics, Vol 203, p 1-60, 2000].

D'autres procédés appropriés pour réduire le degré d'humidité ou la teneur en eau de la composition sont envisageables. De préférence le degré d'humidité est inférieur ou égal à 3% en poids, de préférence inférieur ou égal à 2,5%, de préférence inférieur ou égal à 2%, de préférence inférieur ou égal à 1,5%.

La composition selon l'invention peut être avantageusement soumise à une méthode d'élimination ou d'inactivation des agents infectieux, par exemple par chauffage à sec du lyophilisat.

La composition solide selon l'invention, de préférence sous forme lyophilisée, peut être dissoute dans de l'eau pour préparations injectables (ou « water for injection ou WFI »), pour obtenir une formulation à usage thérapeutique.

De manière préférée, la dissolution du facteur VII peut être réalisée dans l'eau pure, ce qui est avantageux par rapport à des solvants de reconstitution plus complexes comme le solvant avec de l'histidine utilisé dans le produit NovoSeven® RT.

La composition liquide (avant dessiccation) selon l'invention possède une stabilité chimique ou physique d'au moins 3 jours entre 2 et 8 °C.

La composition solide selon l'invention possède une stabilité chimique ou physique sous forme solide supérieure à 24 mois, et généralement jusqu'à au moins 36 mois, à une température inférieure ou égale à 25°C. La formulation injectable reconstituée est également très stable, sa stabilité chimique et physique sous forme liquide étant supérieure à 6 heures à 25°C, de préférence supérieure à 12 heures, de préférence supérieure à 24h, de préférence encore supérieure à une semaine.

La composition pharmaceutique sous forme liquide ou sous forme solide ou la formulation injectable est utile pour le traitement de diverses pathologies.

Un objet de l'invention est une composition pharmaceutique, ou formulation injectable telle que définie plus haut, pour une utilisation dans le traitement d'une hémophilie ou d'un déficit congénital en facteur VII.

Il est également décrit une méthode de traitement d'une hémophilie ou d'un déficit congénital en facteur VII, dans laquelle on administre à un patient nécessitant un tel traitement une quantité efficace de la formulation injectable décrite.

L'hémophilie peut être de type A ou de type B. L'hémophilie de type A est caractérisée par un déficit en facteur VIII alors que l'hémophilie de type B quant à elle, est issue d'un déficit en facteur IX. Le déficit congénital en facteur VII est une maladie hémorragique héréditaire de transmission autosomique récessive rare, due à la diminution ou l'absence du facteur VII de la coagulation.

La formulation injectable peut être administrée par voie parentérale (intraveineuse, sous-cutanée, intramusculaire), en une quantité appréciée par le praticien. L'administration de la forme liquide (avant dessiccation) ou de la forme solide, par toute voie et tout moyen appropriés, n'est pas exclue.

Les exemples et figures suivants illustrent l'invention sans toutefois en limiter la portée.

### Légendes des Figures :

La Figure 1 est un graphe qui montre que l'activité du FVIIa issue de la formulation F3 est préservée après sa reconstitution sous forme liquide, suite à un stockage de 6 jours à 25°C
La figure 2 est un graphe qui montre que les formulations F3, F4 et F7 sous forme solides permettent la préservation l'activité de la protéine (FVIIa) (exprimée par le rapport de FVIIa/FVII:Ag) lors d'un stockage à 40°C pendant 1 mois.

### EXEMPLES:

Le FVII utilisé dans les exemples est obtenu à partir du lait de lapines transgéniques, comme décrit dans la demande de brevet WO2008099077. Il s'agit de facteur VII humain qui est activé au cours de sa purification.

### Exemple 1 : Préparation des formulations :

### 1. 1 : Préparation de formulations liquides F1 à F7 :

Du facteur VII purifié et activé (10-20ml) à une concentration approximative de 0,6mg/ml (formulations F1 à F5) et 0,4mg/ml (formulations F6 et F7), respectivement, a été dialysé pendant 12 heures contre 2 litres de solution tampon, telle que définie dans la ligne correspondante du Tableau 1. Le pH (6,0 ± 0,2) a été ajusté avec soit 1M NaOH soit 1M HCl. Les solutions de FVIIa formulées ont été filtrées et réparties dans des flacons à raison de 0,5ml par flacon. Les flacons ont été ensuite pré-bouchés en utilisant des bouchons en bromobutyle.

### 1.2 : Préparation de formulations lyophilisées issues des formulations liquides F1 à F7 préparées au point 1.1 ci-dessus :

Les flacons ont été lyophilisés selon un cycle prédéfini. Pour permettre de détecter la fin de la dessiccation le lyophilisateur a été équipé d'un capteur d'humidité à capacitance. A la fin du cycle de lyophilisation, les flacons ont été bouchés sous vide et scellés avec des capsules en aluminium.

### 1. 3 : Préparation de la formulation liquide F8 :

Du facteur VII purifié et activé (0,4mg/ml) a été formulé par échange de tampon sur une colonne de gel filtration Superdex 200. La colonne a été initialement équilibrée avec une solution tampon comprenant le citrate trisodique (1,0g/l), de chlorhydrate d'arginine (30g/l) et de l'isoleucine (6,0g/l) (voir Tableau : F8). Le pH (7,0 ± 0,2) a été ajusté. La concentration de l'éluat a été déterminée à environ 0,4mg/ml.

La solution de FVIIa formulée a été filtrée et répartie dans des flacons à raison de 0,5ml par flacon. Ensuite les flacons ont été pré-bouchés en utilisant des bouchons en bromobutyle.

### 1.4 : Préparation de formulation lyophilisée issue de formulation liquide F8 préparée au point 1.3 ci-dessus :

Les flacons ont été lyophilisés selon un cycle prédéfini. Pour suivre la fin de la dessiccation le lyophilisateur a été équipé d'un capteur d'humidité à capacitance. A la fin du cycle de lyophilisation, les flacons ont été bouchés sous vide et scellés avec des capsules en aluminium.

### 1.5 : Préparation de la formulation liquide F9 et lyophilisation:

Du facteur VII purifié et activé (0,4 mg/ml environ) a été formulé par échange de tampon sur une colonne de gel filtration Superdex 200. La colonne a été initialement équilibrée avec une solution tampon comprenant le citrate trisodique (1,0g/l), de chlorhydrate d'arginine (30g/l) et de l'isoleucine (6,0g/l) (voir Tableau : F9). Le pH (7,0 ± 0,2) a été ajusté. De la glycine (1,2 mg/ml) et du chlorhydrate de lysine (1,2 mg/ml) ont été ajoutées. La solution de FVIIa formulée a été filtrée et répartie dans des flacons à raison de 1,0 ml par flacon. Ensuite les flacons ont été pré-bouchés en utilisant des bouchons en bromobutyle.

Les flacons ont ensuite été lyophilisés de la même façon que précédemment et stockés à 40°C pendant 6 mois.

### 1.6 : Préparation des formulations liquides F10 et F11 et lyophilisation:

Du facteur VII purifié et activé à une concentration approximative de 1,0 mg/ml (F10) et 0,8 mg/ml (F11), respectivement, a été formulé par dialyse en boudin avec le tampon tel que définit dans les tableaux 3 et 4 (voir tableau : F10 et F11). Le pH (7,0 ± 0,2) a été ajusté.

Les solutions de FVIIa formulées ont été filtrées et réparties dans des flacons à raison de 1,0 ml par flacon. Ensuite les flacons ont été pré-bouchés en utilisant des bouchons en bromobutyle.

Les flacons ont ensuite été lyophilisés de la même façon que précédemment.

La formulation F10 a été conservée dans les conditions suivantes :
- Liquide à 5°C pendant 144 heures puis à 25°C pendant 6 heures,
- Cycle de congélation et décongélation (4 cycles consécutifs),
- Lyophilisée et stockée à 25°C et 40°C durant 6 mois,
- Congelée à < - 70°C pendant 6 mois.

La formulation F11 a été conservée dans les conditions suivantes :
- Liquide pendant 72 heures à 5°C plus 6 heures supplémentaires à 25°C,
- Lyophilisée et stockée à 25°C et 40°C,
- Congelée à < - 70°C dans le tampon avant l'étape de dialyse : Trométamol (2,42 mg/ml), NaCl (8,77 mg/ml) et mannitol (30 mg/ml).

### Exemple 2 : Tests des formulations

### Matériels et méthodes :

### 2.1. Mesures de diffusion dynamique de la lumière, (Dynamic Light Scattering), ci-après « DLS ».

500 microlitres de chaque échantillon issus de la lyophilisation ont été placés dans une microcuvette (Plastibrand®, Wertheim, Allemagne), qui a été transférée dans un appareil Zetasizer Nano (Malvern Instruments, Worcestershire, UK). Cet appareil fonctionne avec un laser à 633nm He-Ne à 4mM, et une technique non invasive de rétrodiffusion « Non-Invasive Backscatter » ou NIBS.

La distribution de taille des populations monomériques de la protéine par intensité et volume a été calculée à partir du logiciel Dispersion Technology Software de Malvern (version 4.00). Les indices de réfraction du matériau et du dispersant ont été définis à 1,33 et 1,45 respectivement. La température durant les mesures a été contrôlée et fixée à 20°C. Des paramètres de qualité pour les formulations testées incluent le diamètre de la population de protéines sous forme de monomères et l'intensité de diffusion de la population de protéines.

### 2.2. Inspection visuelle des formulations reconstituées.

La formulation à tester a été reconstituée à partir de la formulation lyophilisée avec 0,5g d'eau pour préparations injectables. Le solvant a été injecté dans le flacon du lyophilisat à l'aide d'une seringue via le bouchon

Après dissolution complète du gâteau, le produit reconstitué a été examiné selon la méthode d'inspection visuelle relative à la Pharmacopée Européenne (Methods of Analysis - Pharmaceutical Technical Procedures - Particulate contamination-visible particules - paragraphe 2.9.20).

Les formulations testées ont été classées de manière semi-quantitative, selon leur degré de contamination par des particules visibles :
- = aucune particule visible
ε = quelques rares particules visibles
+ = Particules visibles en faible quantité de particules visibles
++ = grande quantité de particules visibles
+++ = très grande quantité de particules visibles

Tous les tests ont été menés de manière indépendante, et par trois opérateurs différents.

### 2.3. Test sur filtre pour la détection des agrégats.

La méthode utilisée est tirée de l'article [Li et al, A simple method for the détection of insoluble aggregates in protein formulations, Journal of Pharmaceutical Sciences Vol 96 (7), p1840-1843, 2007].

Un filtre membrane stérile Millex® GV 0,2µm a été rincé avec 3ml d'eau puis le même volume de solution tampon. Ensuite 0,5ml de solution protéique (FVIIa) à analyser a été filtré sur ce filtre. Puis le filtre a été rincé de nouveau avec des volumes égaux d'eau (3ml) pour préparations injectables puis de solution tampon (3ml). Les fractions de protéines retenues par le filtre ont été colorées par l'addition de 2ml de solution de coloration (« Reversible Protein Detection KIT » de SIGMA). La solution de coloration a été maintenue en contact avec la membrane de filtration pendant 5 min avant écoulement. Puis la membrane a été rincée de nouveau comme décrit ci-dessus. Les formulations testées ont été classifiées de manière semi-quantitative, selon leur degré de particules colorées observées sur le filtre, provenant de protéines :
- = Aucune particule visible détectée, provenant de protéines.
ε = Quelques rares particules visibles détectées, provenant de protéines
+ = Particules visibles en faible quantité détectées, provenant de protéines
++ = Particules visibles en grande quantité détectées, provenant de protéines
+++ = Particules visibles en très grande quantité détectées, provenant de protéines

### 2.4. Détermination de la température de transition vitreuse (Tg).

La température de transition vitreuse a été déterminée par un thermoanalyseur différentiel à balayage DSC 7 (Perkin Elmer) calibré à l'aide d'indium (de température de fusion (Tm) 156,6°C) et de n-octadécane (Tm 38,2°C). Les échantillons ont subi des températures de - 50 à 138°C à la vitesse de 20°C/min. L'hélium a été utilisé pour mener les expériences à une température inférieure à la température ambiante. La température de transition vitreuse a été prise au point médian du changement endothermique dans la chaleur spécifique apparente. Deux mesures ont été réalisées et la moyenne donne la Tg.

### 2.5. Détermination du rapport FVIIa/FVII :Ag

L'activité du Facteur VII dans la composition est évaluée par le rapport entre la quantité de Facteur VIIa déterminée par un test de coagulation et la quantité de Facteur VII déterminée par immunoréactivité avec des anticorps anti-FVII.

### Dosage du Facteur VII (désigné ici « Facteur VII antigène » ou « FVIIAg »):

Le FVII est dosé par méthode immunoenzymatique (ELISA) avec des réactifs commerciaux (Diagnostica Stago). Brièvement, le Facteur VII à doser est capté par un anticorps anti-Facteur VII humain immobilisé sur une phase solide. Le Facteur VII fixé est ensuite reconnu par un immuno-conjugué à la peroxydase. Le taux de peroxydase liée est mesuré par son activité sur le substrat ortho-phénylènediamine en présence d'eau oxygénée. L'intensité de la coloration, après arrêt de la réaction par un acide fort, est fonction de la quantité de facteur VII présente initialement dans l'échantillon.

### Dosage du Facteur FVII sous forme activée :

Le Facteur FVII activé (FVIIa) est mesuré par méthode chronométrique avec des réactifs commerciaux (Diagnostica Stago). Le Facteur tissulaire soluble recombinant (rsTF) possède une fonction cofacteur du Facteur VIIa et permet en présence de phospholipides et de calcium la coagulation du plasma. Dans ce système, le temps de coagulation obtenu sera fonction de la quantité de Facteur VII contenue dans l'échantillon à tester. Le rsTF n'active pas le Facteur VII en Facteur VIIa; par conséquent le Facteur VII contenu dans le plasma n'interfère pas dans le dosage.

### 2.6 : Distribution de la taille moléculaire (DTM) :

La détermination de la distribution de la taille moléculaire s'effectue grâce à un système de chromatographie équipé avec une pompe, un injecteur thermostaté, un détecteur UV, d'un système d'acquisition informatique en utilisant une colonne Superdex Tricorn 200 10/300 GL (GE Healthcare, réf. 17-5175-01). La phase mobile se compose de 0,01M tampon phosphate, 0,138M chlorure de sodium et 0,0027M de chlorure de potassium à pH 7,4. Son débit s'élève à 0.4 ml/min. Pour l'analyse 100 µl de l'échantillon sont injectés. La détection par UV est réalisée à 280 nm.

### 2.7 : SDS-PAGE (réduit/non réduit) :

La qualité des échantillons au niveau du taux des agrégats covalents et des fragments a été évaluée par analyse des différences liées à la masse des protéines par migration électrophorétique SDS-PAGE, en conditions non-réductrices et en conditions réductrices sur un système Novex en utilisant des gels en tampon acide 2-(*N-*morpholino)ethanesulfonic ou MES (Invitrogen). Une quantité correspondante à 2 µg en protéine a été déposée. La révélation des protéines s'est effectuée par coloration Coomassie bleu et/ou nitrate d'argent.

### 2.8 : IEF

La séparation des différentes isoformes du FVII selon leur point isoélectrique a été réalisée par focalisation isoélectrique (IEF). La migration s'est effectuée sur un Focugel 3-10 ETC (Gelcompany) sur un système Multiphor en conditions natives (non réductrices et non dénaturantes). Le produit a été soumis à un dessalage sur une unité de filtration (taille d'exclusion 10 kDa). Après avoir déterminé la concentration du produit par DO à 280 nm, 30 µg ont été déposés sur le gel. La comparaison des populations contenues dans le produit avec les deux différentes standards de pI (standard pI 5,5-10,5 de GE et standard pI 5,4-5,9-6,6 de Sigma, mis des standards individuels) après une coloration par CBB-G250 (Coomassie Brilliant Blue G250) a permis l'identification du pI des isoformes du FVII (quantification par logiciel Quantity one, BioRad).

### Résultats :

### 1. Formulations F1 à F8

Dans le tableau 1 ci-dessous, les formulations F1, F2 sont données à titre comparatif. Parmi les compositions selon l'invention (F3 à F8), les formulations F3, F4 et F7 sont préférées.

D'après ce tableau, les formulations F4, F5 et F7 montrent un pourcentage d'intensité de la population monomérique de la protéine (respectivement 58 %, 61% et 55%), supérieure à celles obtenues dans les formulations NovoSeven® (F1) (9%) et NovoSeven RT® (F2) (27%). Ces résultats traduisent une augmentation de la stabilité du produit sous forme solide et la présence de monomères permet de diminuer les risques de réactions immunologiques.

En ce qui concerne le rapport FVIIa/FVII:Ag, les formulations F3, F4 et F8 présentent des rapports FVIIa/FVII:Ag avant lyophilisation et après reconstitution sous forme liquide plus élevés que ceux obtenus avec des formulations telles que celles de NovoSeven® (F1) et de NovoSeven RT® (F2), ce qui traduit que la protéine (FVIIa) contenue dans la composition selon l'invention est plus active. On peut observer un rapport FVIIa/FVII:Ag avant lyophilisation de 17 pour F3 et 23 pour F8 contre 14 pour ceux obtenus avec les formulations NovoSeven® (F1) et NovoSeven RT®, ainsi qu'un rapport FVIIa/FVII:Ag après reconstitution sous forme liquide de 19 pour F3 et 20 pour F8 contre 15 pour ceux obtenus avec les formulations NovoSeven® (F1) et NovoSeven RT®,

Pour ce qui concerne les expériences réalisées sur la température de transition vitreuse (Tg), les formulations F3 à F8 selon l'invention montrent une augmentation de la température de transition vitreuse. On obtient notamment pour la formulation F3 une température de transition vitreuse égale à 75°C, et de 93 °C pour la formulation F7. Ces résultats montrent que la protéine (FVIIa) est avantageusement stable dans ces formulations. En effet, une Tg élevée permet une stabilité améliorée de la protéine en réduisant la mobilité et la réactivité du principe actif. De plus, ces résultats montrent bien que les produits selon l'invention peuvent être conservés hors réfrigérateur contrairement au produit NovoSeven® de la société NovoNordisk.

En ce qui concerne le test sur filtre pour la détection des agrégats, les résultats indiquent que les formulations F3 à F8 selon l'invention présentent peu d'agrégats voire une absence totale d'agrégats pour les formulations F4, F7 et F8.

Comme le montre la Figure 1, l'activité du FVII issu de la formulation F3 (sous forme liquide après reconstitution) est encore satisfaisante au bout de 6 jours, avec une activité d'au moins 80%.

Comme le montre la Figure 2, l'activité du FVII issu de la formulation F3 (sous forme solide) est encore satisfaisante au bout d'un mois, avec une activité d'au moins 80% pour la formulation F3, au moins 90% pour la formulation F4.

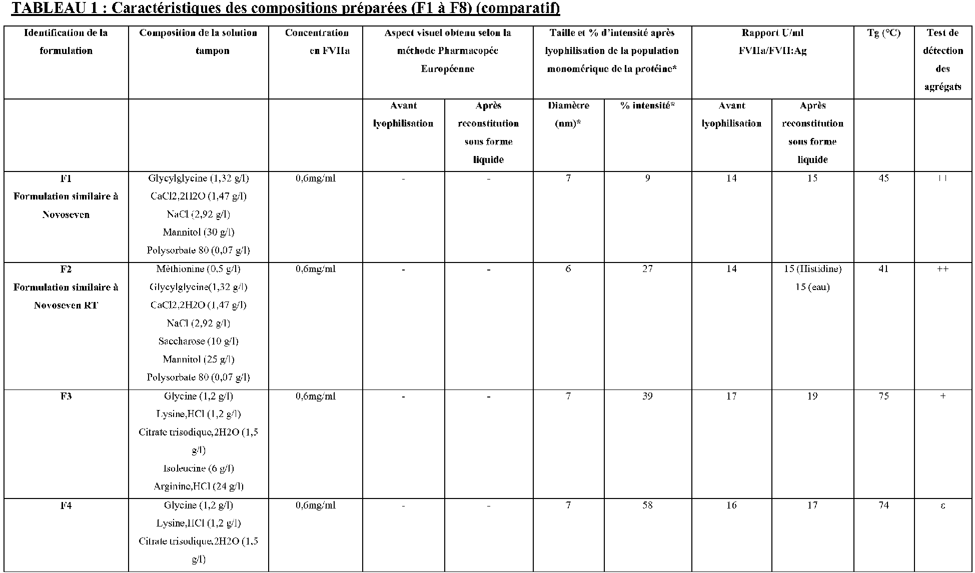

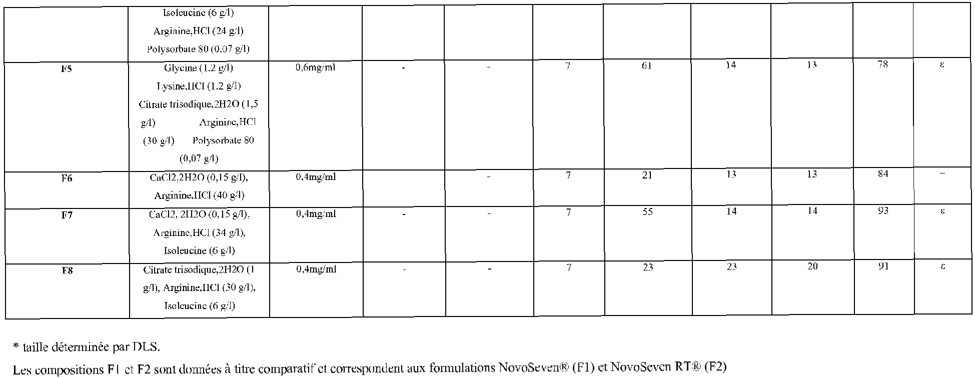

### 2. Formulations F9 à F11 - Etudes de stabilité

Les résultats des tests réalisés sur ces formulations sont présentés dans les tableaux 2 à 4 ci-dessous.

### 2.1 Etude de stabilité : Formulation F9

La formulation F9 reste stable également après 6 mois à 40°C, sous forme lyophilisée. De plus, les résultats montrent que le FVII ne présente pas de signe de dégradation évident après 6 mois à 40°C.

### 2.2 Etude de stabilité : Formulation F10

Les études réalisées sur la formulation F10 (Tableau 3) ont permis de montrer que le FVIIa est stable pendant 48 heures à 5°C, sous forme liquide.

Comme on peut le voir également dans le Tableau 2, la formulation F10 reste stable après quatre cycles de congélation/décongélation et les résultats obtenus témoignent d'une absence de dégradation du FVII.

La formulation F10 reste stable également après 6 mois à 25°C ou à 40°C, sous forme lyophilisée. Les résultats de cette étude montrent que le FVII n'a subit aucune dégradation. La formulation F10 (non lyophilisée) reste stable également après 6 mois congelée à une température inférieure à -70°C,. Les résultats obtenus prouvent que le FVII n'a subit aucune dégradation.

### 2.3 Etude de stabilité : Formulation F11

La formulation F11 (Tableau 4) est stable après 6 mois à 25°C ou à 40°C, sous forme lyophilisée. Les résultats de cette étude montrent que le FVII n'a subit aucune dégradation. La formulation F11 est stable également après 6 mois congelée à une température inférieure à -70°C, sous forme lyophilisée. Les résultats obtenus prouvent que le FVII n'a subit aucune dégradation.

L'étude de stabilité sous forme liquide de la formulation F11 montre que le FVII reste stable pendant les 72 heures à 5°C plus les 6 heures à 25°C.

**TABLEAU 2 : Caractéristiques des compositions préparées (F9) (comparatif)**

| **Formulation F9** | **Aspect visuel obtenu selon la méthode Pharmacopée Européenne** | | **% d'intensité de la population contenant la protéine dans sa forme monomérique (mesurer par DLS)** | | **Rapport U/ml FVIIa/FVII;Ag** | | **Test de détection des agrégats** | **Analyses DTM (% monomères)** | | **SDS-PAGE et IEF** |
|---|---|---|---|---|---|---|---|---|---|---|
| | **T=0 (Après lyophilisation)** | **T=6 mois** | **T=0 (Après lyophilisation)** | **T=6 mois** | **T=0 (Après lyophilisation)** | **T=6 mois** | **T=6mois** | **T=0** | **T=6 mois** | |
| **Lyophilisée à 40°C** | - | - | 61% | 52% | 16 | 16 | - | 86% | 90% | Profils comparables de T=0 et T=6 mois |

**TABLEAU 3 : Caractéristiques des compositions préparées (F10) (comparatif)**

| **Formulation F10** | **Aspect visuel obtenu selon la méthode Pharmacopée Européenne** | | **% d'intensité de la population contenant la protéine dans sa forme monomérique (mesurer par DLS)** | | **Rapport U/ml FVIIa/FVII;Ag** | | **Test de détection des agrégats** | **Analyses DTM (% monomères)** | | **SDS-PAGE et IEF** |
|---|---|---|---|---|---|---|---|---|---|---|
| | **T=0** | **T=48h à 5°C** | **T=0** | **T=48h à 5°C** | **T=0** | **T=48h à 5°C** | **T=48h à 5°C** | **T=0** | **T=48h à 5°C** | |
| Liquide à 5°C | - | - | 73% | 70% | 16 | 14 | ε | 92% | 88% | Profils comparables de T=0 et T=48H 5°C |
| | **T=0 (Après lyophilisation)** | **T=6 mois** | **T=0 (Après lyophilisation)** | **T=6 mois** | **T=0 (Après lyophilisation)** | **T=6 mois** | **T=6mois** | **T=0** | **T=6 mois** | |
| Lyophilisée à 25°C | - | - | 55% | 71% | 16 | 16 | ε | 91% | 93% | Profils comparables de T=0 et T=6 mois |
| Lyophilisée à 40°C | - | - | 55% | 65% | 16 | 15 | ε | 91% | 92% | Profils comparables de T=0 et T=6 mois |
| Congélation à <-70°C | - | - | 73% | 87% | 16 | 16 | ε | 92% | 92% | Profils comparables de T=0 et T=6 mois |
| | **T=0** | **T=4 cycles** | **T=0** | **T=4 cycles** | **T=0** | **T=4 cycles** | **T=4 cycles** | **T=0** | **T=4 cycles** | |
| 4 cycles de congélation/décongélation | - | - | 73% | 63% | 16 | 13 | ε | 92% | 89% | Profils comparables de T=0 et T= 4 cycles |

**TABLEAU 4 : Caractéristiques des compositions préparées (F11) (comparatif)**

| **Formulation F11** | **Aspect visuel obtenu selon la méthode Pharmacopée Européenne** | | **% d'intensité de la population contenant la protéine dans sa forme monomérique (mesurer par DLS)** | | **Rapport U/ml FVIIa/FVII;Ag** | | **Test de détection des agrégats** | **Analyses DTM (% monomères)** | | **SDS-PAGE et IEF** |
|---|---|---|---|---|---|---|---|---|---|---|
| | **T=0** | **T=72h à 5°C + 6h à 25°C** | **T=0** | **T=72h à 5°C + 6h à 25°C** | **T=0** | **T=72h à 5°C + 6h à 25°C** | **T=72h à 5°C + 6h à 25°C** | **T=0** | **T=72h à 5°C + 6h à 25°C** | |
| Liquide à 5°C | - | - | 65% | 66% | 15 | 21 | NR | NR | NR | NR |
| | **T=0 (Après lyophilisation)** | **T=6 mois** | **T=0 (Après lyophilisation)** | **T=6 mois** | **T=0 (Après lyophilisation)** | **T=6 mois** | **T=6mois** | **T=0** | **T=6 mois** | |
| Lyophilisée 25°C | - | - | 65% | 66% | 19 | 20 | ε | 98% | 93% | Profils comparables de T=0 et T=6 mois |
| Lyophilisée 40°C | - | - | 65% | 55% | 19 | 19 | ε | 98% | 97% | Profils comparables de T=0 et T=6 mois |
| Congélation -70°C | - | - | 87% | 87% | 19 | 18 | ε | NR | 97% | Profils comparables de T=0 et T=6 mois |

| | | | | | | | | | | |
|---|---|---|---|---|---|---|---|---|---|---|
| **= non réalisé** | | | | | | | | | | |

## Revendications

1. Composition pharmaceutique sous forme liquide ou sous forme solide, dépourvue de mannitol et de saccharose, comprenant :
- facteur VII,
- arginine, éventuellement sous forme de chlorhydrate ;
- isoleucine ;
- lysine , éventuellement sous forme de chlorhydrate ;
- glycine ;
- citrate trisodique ou chlorure de calcium ;
- et le cas échéant du polysorbate 80 ou polysorbate 20.

2. Composition selon la revendication 1, ladite composition étant dépourvue de tout sucre, de polyol ou de méthionine.

3. Composition selon l'une des revendications 1 ou 2, ladite composition étant en outre dépourvue de glycylglycine.

4. Composition selon l'une des revendications 1 à 3, dans laquelle le facteur VII est sous forme activée (FVIIa).

5. Composition selon la revendication 1, comprenant :
- facteur VII, de préférence sous forme de facteur VIIa ;
- de 10 à 40 g/l d'arginine, éventuellement sous forme de chlorhydrate ;
- de 4,2 à 6,6 g/l d'isoleucine ;
- de 0,6 à 1,8 g/l de lysine , éventuellement sous forme de chlorhydrate ;
- de 0,6 à 1,8 g/l de glycine ;
- de 1 à 2 g/l de citrate trisodique dihydraté ou de 0 à 0,2 g/L de chlorure de calcium dihydraté;
- et le cas échéant de 0 à 0,5 g/l de polysorbate 80.

6. Composition selon la revendication 7, comprenant:
- du facteur VII, de préférence sous forme de facteur VIIa, à 0,2 à 2g/l,
- du chlorhydrate d'arginine à 24 g/l,
- de l'isoleucine à 6g/l,
- du citrate trisodique dihydraté à 1,5g/l,
- de la glycine à 1,2g/l,
- du chlorhydrate de lysine à 1,2g/l.
- et /ou du polysorbate 80 à 0,07g/l.

7. Composition selon la revendication 1, comprenant:
- facteur VII, de préférence sous forme de facteur VIIa ,à 0,2 à 2g/l,
- du chlorhydrate d'arginine à 34 g/l,
- du chlorure de calcium dihydraté à 0,15 g/l,
- de l'isoleucine à 6g/l.

8. Composition solide lyophilisée susceptible d'être obtenue à partir d'une composition selon l'une des revendications 1 à 7.

9. Procédé de préparation d'une composition pharmaceutique selon l'une quelconque des revendications 1 à 8, ledit procédé comprenant le mélange du FVII avec une solution tampon, ajustement du pH si nécessaire, filtration puis dessiccation si nécessaire pour obtenir la forme solide.

10. Procédé de préparation d'une formulation injectable à usage thérapeutique, ledit procédé comprenant la dissolution d'une composition solide telle que définie à l'une des revendications 1 à 8, dans de l'eau pour préparation injectable.

11. Formulation injectable susceptible d'être obtenue par le procédé de la revendication 10.

12. Composition pharmaceutique telle que définie à l'une des revendications 1 à 8, ou formulation injectable telle que définie à la revendication 11, pour une utilisation dans le traitement d'une hémophilie ou d'un déficit congénital en facteur VII.

## Patentansprüche

1. Pharmazeutische Zusammensetzung in flüssiger oder fester Form, frei von Mannit und Saccharose, umfassend
- Faktor VII;
- Arginin, eventuell in Form eines Hydrochlorids;
- Isoleucin;
- Lysin, eventuell in Form eines Hydrochlorids;
- Glycin;
- Trinatriumcitrat oder Calciumchlorid;
- und gegebenenfalls Polysorbat 80 oder Polysorbat 20.

2. Zusammensetzung gemäß Anspruch 1, wobei besagte Zusammensetzung frei von jeglichem Zucker, Polyol oder Methionin ist.

3. Zusammensetzung gemäß einem der Ansprüche 1 oder 2, wobei besagte Zusammensetzung außerdem frei von Glycylglycin ist.

4. Zusammensetzung gemäß einem der Ansprüche 1 bis 3, wobei der Faktor VII in aktivierter Form (FVIIa) vorliegt.

5. Zusammensetzung gemäß Anspruch 1, umfassend:
- Faktor VII, vorzugsweise in Form von Faktor VIIa;
- 10 bis 40 g/l Arginin, eventuell in Form eines Hydrochloride;
- 4,2 bis 6,6 g/l Isoleucin;
- 0,6 bis 1,8 g/l Lysin, eventuell in Form eines Hydrochlorids;
- 0,6 bis 1,8 g/l Glycin;
- 1 bis 2 g/l Trinatriumcitrat-Dihydrat oder 0 bis 0,2 g/l Calciumchlorid-Dihydrat;
- und gegebenenfalls 0 bis 0,5 g/l Polysorbat 80.

6. Zusammensetzung gemäß Anspruch 7, umfassend
- Faktor VII, vorzugsweise in Form von Faktor VIIa, mit 0,2 bis 2 g/l;
- Arginin-Hydrochlorid mit 24 g/l;
- Isoleucin mit 6 g/l;
- Trinatriumcitrat-Dihydrat mit 1,5 g/l;
- Glycin mit 1,2 g/l;
- Lysin-Hydrochlorid mit 1,2 g/l;
- und/oder Polysorbat 80 mit 0,07 g/l.

7. Zusammensetzung gemäß Anspruch 1, umfassend
- Faktor VII, vorzugsweise in Form von Faktor VIIa, mit 0,2 bis 2 g/l;
- Arginin-Hydrochlorid mit 34 g/l;
- Calciumchlorid-Dihydrat mit 0,15 g/l;
- Isoleucin mit 6 g/l.

8. Lyophilisierte feste Zusammensetzung, die aus einer Zusammensetzung gemäß einem der Ansprüche 1 bis 7 erhalten werden kann.

9. Verfahren zur Herstellung einer pharmazeutischen Zusammensetzung gemäß einem der Ansprüche 1 bis 8, wobei besagtes Verfahren das Mischen von FVII mit einer Pufferlösung, Einstellen des pH, falls erforderlich, Filtration und anschließend Trocknen, falls erforderlich, umfasst, um die feste Form zu erhalten.

10. Verfahren zur Herstellung einer injizierbaren Formulierung zur therapeutischen Verwendung, wobei besagtes Verfahren das Lösen einer wie in einem der Ansprüche 1 bis 8 definierten festen Zusammensetzung in Wasser umfasst, um ein injizierbares Präparat zu erhalten.

11. Injizierbare Formulierung, die mit dem Verfahren nach Anspruch 10 erhalten werden kann.

12. Pharmazeutische Zusammensetzung, wie in einem der Ansprüche 1 bis 8 definiert, oder injizierbare Formulierung, wie in Anspruch 11 definiert, für eine Verwendung in der Behandlung einer Hämophilie oder eines angeborenen Mangels an Faktor VII.

## Claims

1. A pharmaceutical composition in liquid form or in solid form, being free of mannitol and of sucrose, comprising:
- factor VII ;
- arginine, optionally in hydrochloride form ;
- isoleucine ;
- lysine, optionally in hydrochloride form ;
- glycine ;
- trisodium citrate or calcium chloride ;
- and, where appropriate, polysorbate 80 or polysorbate 20.

2. The composition according to claim 1, said composition being free of any sugar, of polyol or of methionine.

3. The composition according to one of claims 1 or 2, said composition further being free of glycylglycine.

4. The composition according to one of claims 1 to 3, wherein factor VII is in activated form (FVIIa).

5. The composition according to claim 1, comprising :
- factor VII, preferably in the form of factor VIIa ;
- from 10 to 40 g/l of arginine, optionally in hydrochloride form ;
- from 4.2 to 6.6 g/l of isoleucine ;
- from 0.6 to 1.8 g/l of lysine, optionally in hydrochloride form ;
- from 0.6 to 1.8 g/l of glycine ;
- from 1 to 2 g/l of trisodium citrate dihydrate or from 0 to 0.2 g/l of calcium chlorine dihydrate ;
- and, where appropriate, from 0 to 0.5 g/l of polysorbate 80.

6. The composition according to claim 7, comprising :
- factor VII, preferably in the form of factor VIIa, at 0.2 to 2 g/l,
- arginine hydrochloride at 24 g/l,
- isoleucine at 6 g/l,
- trisodium citrate dihydrate at 1.5 g/l,
- glycine at 1.2 g/l,
- lysine hydrochloride at 1.2 g/l,
- and/or polysorbate 80 at 0.07 g/l.

7. The composition according to claim 1, comprising :
- factor VII, preferably in the form of factor VIIa, at 0.2 to 2 g/l,
- arginine hydrochloride at 34 g/l,
- calcium chlorine dihydrate at 0.15 g/l,
- isoleucine at 6 g/l.

8. A lyophilized solid composition obtainable from a composition according to one of claims 1 to 7.

9. A method for preparing a pharmaceutical composition according to any one of claims 1 to 8, said method comprising mixing the FVII with a buffer solution, adjusting the pH if necessary, filtering, and then desiccating if necessary so as to obtain the solid form.

10. A method for preparing an injectable formulation for therapeutic use, said method comprising dissolving a solid composition as defined in one of claims 1 to 8, in water for injection.

11. An injectable formulation obtainable by the method of claim 10.

12. The pharmaceutical composition as defined in one of claims 1 to 8, or the injectable formulation as defined in claim 11, for use in treating haemophilia or congenital factor VII deficiency.
